# FASCICULE DE BREVET EUROPEEN

(11) **EP 2 349 003 B1**
(45) Date de publication et mention de la délivrance du brevet: **20.03.2013**
(21) Numéro de dépôt: 09740686.2
(22) Date de dépôt: 22.10.2009
(51) Int. Cl.: A61B 5/22

(54) **PROCEDE ET DISPOSITIF PERMETTANT A UN ATHLETE DE DETERMINER PUIS DE CONTROLER LA VITESSE DE DEPLACEMENT D'UNE MASSE**
VERFAHREN UND VORRICHTUNG, DAS BZW. DIE ES EINEM SPORTLER ERMÖGLICHT, DIE MASSENVERSCHIEBERATE ZU BESTIMMEN UND DANN ZU KONTROLLIEREN
METHOD AND DEVICE ENABLING AN ATHLETE TO DETERMINE AND THEN CONTROL THE RATE OF DISPLACEMENT OF A MASS

(30) Priorité: 22.10.2008 CH 16632008
(43) Date de publication de la demande: 03.08.2011
(73) Titulaire: Myotest SA, 1950 Sion (CH)
(72) Inventeur: FLACTION, Patrick, CH-1965 Chandolin-près-Savièse (CH)
(74) Mandataire: P&TS SA (AG, Ltd.)
(86) Numéro de dépôt international: PCT/EP2009/063916
(87) Numéro de publication internationale: WO 2010/046448

(56) Documents cités:
- EP-A1- 1 834 583
- WO-A1-2007/036611
- WO-A2-2008/030484
- JP-A- 2006 122 343
- US-A1- 2007 219 059
- US-A1- 2008 090 703

## Description

### Domaine technique

La présente invention concerne un procédé et un dispositif permettant à un athlète de déterminer puis de contrôler la vitesse de déplacement d'une masse.

### Etat de la technique

Il existe de nombreux documents décrivant des accéléromètres pour la mesure de performances sportives. La plupart des accéléromètres existants sont destinés à des exercices de longue durée, par exemple pour évaluer la distance parcourue ou le nombre de calories dépensées pendant une séance de jogging ou une randonnée cycliste. Il existe aussi des dispositifs assez similaires pour détecter les chutes de personnes âgées, le temps qu'elles passent assis, debout ou couchées, etc.

US5788655 (Omron) décrit un appareil destiné à être fixé sur le corps et muni d'un accéléromètre et d'un afficheur LCD. L'appareil mesure en permanence les déplacements du porteur pour déterminer son niveau d'activité physique et des d'autres grandeurs dépendant du métabolisme, par exemple la consommation de calorie journalière de l'utilisateur. Ce type d'appareil est utile pour mesurer de manière plus objective le niveau de sédentarité de patients. Il est cependant inadapté à l'entraînement musculaire et à la mesure d'efforts brefs, et ne permet pas de déterminer par exemple la puissance maximale d'un groupe musculaire de l'athlète.

WO2005074795 (Nokia) décrit un terminal de mesure muni d'un accéléromètre et attaché sur le corps d'un athlète. Les données de mesure sont évaluées pour fournir une grandeur représentative de l'intensité de l'effort fourni. A nouveau, le but est de déterminer le niveau d'activité sur une longue période, par exemple une journée ou une semaine.

US2006191335 décrit un accéléromètre portatif destiné notamment à mesurer la force musculaire des jambes à l'aide d'exercices de marche.

WO03/032826 (Philips) décrit un système comparable et muni d'un accéléromètre à trois axes pour déterminer le niveau d'activité physique d'un patient. Le dispositif proposé affiche des grandeurs telles que le taux métabolique quotidien, la dépense énergétique quotidienne ou la dépense d'énergie induite par l'exercice. Cet appareil permet donc de mesurer des accélérations sur une période de plusieurs heures, voire de plusieurs jours.

Aucun de ces dispositifs ne permet d'obtenir des paramètres physiologiques musculaires de base comme la force, la vitesse ou la puissance d'un groupe musculaire. Ils sont essentiellement destinés aux sports d'endurance, par exemple au jogging, et pas ou mal adaptés à l'entraînement spécifique de la force, de la puissance ou de la vitesse de contraction d'un muscle ou d'un groupe musculaire. A défaut de connaitre la masse déplacée, nombre de dispositifs ne permettent pas de déduire la force ou la puissance de l'athlète. Même lorsque cette masse est connue, beaucoup de dispositifs visent avant tout à calculer la distance parcourue, la vitesse moyenne ou la dépense énergétique, mais ne permettent pas de déterminer directement la capacité musculaire d'un groupe musculaire de l'athlète, c'est-à-dire la vitesse à laquelle ce groupe musculaire peut déplacer une masse donnée.

US5474083 décrit un système destiné à surveiller les mouvements de levé de charge par un patient. Le système emploie des électrodes pour mesurer l'activité des muscles du patient au cours du mouvement, ainsi qu'un détecteur de mouvement de la charge. Une alarme est déclenchée en cas de mouvement inapproprié. Ce système est utile pour empêcher les accidents provoqués par le lever de charges incorrect, ou pour exercer des personnes à soulever des charges sans se blesser. Il est cependant inapproprié à la mesure de la performance musculaire du sportif. Par ailleurs, l'usage d'électrodes rend son emploi peu pratique.

US6148280 (Virtual Technologies) décrit un dispositif muni d'accéléromètres et de gyroscopes disposés sur tout le corps d'un athlète. Les données fournies par plusieurs capteurs sont transmises à un ordinateur personnel qui permet d'analyser la trajectoire et d'autres caractéristiques du mouvement. Ce système est complexe, car il met en oeuvre plusieurs capteurs, y compris des goniomètres coûteux, relativement fragiles. La connexion des capteurs entre eux et vers l'ordinateur externe renchérit le dispositif, et rend son installation malaisée. Il est adapté à l'entraînement de mouvements précis, par exemple un swing de golf, mais ne permet pas de déterminer directement la capacité musculaire développée par le sportif au cours de ce mouvement.

DE446302 décrit un accéléromètre utilisé dans les sports de combat pour la mesure de l'accélération de la surface de frappe. L'appareil n'est pas portable et convient uniquement aux sports de combat tels que boxe, karaté, etc. Un ordinateur externe doit être employé pour évaluer les résultats de mesure et les afficher. Il n'est pas programmable et ne peut être utilisé que pour un seul type d'exercice.

WO2007036611 (Oulun Seudun Ammattikorkeakoulu) décrit un bracelet muni d'un accéléromètre pour mesurer la force musculaire lors de levers de poids.

La demande WO07107491 décrit un accéléromètre portatif permettant d'évaluer la capacité musculaire d'un athlète ou d'un patient à l'aide de tests courts. Ces tests permettent de déterminer la puissance maximale de l'athlète et de sélectionner la charge optimale avec laquelle doit s'exercer un athlète pour maximiser la puissance déployée lors de l'exercice.

Ce dispositif ne fournit cependant aucune indication à l'athlète quant à la vitesse à laquelle il doit déplacer cette charge pour obtenir les résultats désirés. Par ailleurs, les recommandations ne dépendent pas des objectifs qualitatifs de l'athlète, ni des performances qu'il souhaite améliorer. Par exemple, ce dispositif ne permet pas de distinguer entre un athlète souhaitant améliorer sa vitesse et un autre athlète souhaitant améliorer sa puissance. Enfin, ce dispositif est destiné uniquement à des tests courts, à l'aide d'une série d'exercices imposée par le dispositif (par exemple 5 sauts, 5 levers, etc) ; une erreur est générée si l'athlète effectue des mouvements différents, ou un nombre de mouvements différents de ceux prescrits par le test. Ce dispositif est donc inadapté à la mesure de paramètres physiologiques au cours de l'entraînement, par exemple lorsque le nombre de mouvements est variable.

D'autres accéléromètres employés pour la mesure de paramètres musculaires sont aussi décrits dans US 2008 090703, dans GB2422790 et dans WO20005055815.

### Bref résumé de l'invention

Il existe donc un besoin dans l'état de la technique pour un procédé et un dispositif permettant d'améliorer l'efficacité et la qualité des plans d'entraînements de tout athlète dans le milieu sportif.

Il existe aussi un besoin pour un nouvel accéléromètre destiné à la pratique sportive et qui permet de mesurer la capacité musculaire d'un muscle ou d'un groupe musculaire donné.

Il existe aussi un besoin pour un accéléromètre et pour un procédé permettant à un athlète de déterminer des consignes d'entraînement, c'est-à-dire des valeurs pour la masse à déplacer, la vitesse de déplacement et/ou le nombre de déplacements ou de séries de déplacement, afin d'améliorer la capacité musculaire d'un muscle ou d'un groupe musculaire spécifique. Par capacité musculaire, on entend dans la présente demande la capacité à déplacer une masse donnée à une vitesse donnée, un nombre de fois donné.

Il existe également un besoin pour un accéléromètre et pour un procédé permettant de contrôler le suivi de ces consignes lors de l'entraînement, et permettant d'adapter ces consignes aux progrès et aux mesures lors de l'entraînement.

Selon l'invention, ces buts sont atteints notamment au moyen d'un procédé d'entraînement musculaire d'un athlète selon la revendication indépendante 1.

Selon un aspect, le procédé permet à l'athlète de déterminer la masse optimale à soulever pour chaque type d'exercice, en fonction des objectifs de l'athlète, ainsi que la vitesse à laquelle cette masse doit être soulevée. Lors de l'entraînement, le dispositif de l'invention permet de vérifier si la masse est effectivement soulevée à la bonne vitesse, puis d'adapter les consignes de masse et de vitesse pour les futurs entraînements en fonction de valeurs d'accélération mesurées.

### Brève description des figures

Des exemples de mise en oeuvre de l'invention sont indiqués dans la description illustrée par les figures annexées dans lesquelles :

La figure 1 illustre de manières schématiques différentes phases des mouvements d'un athlète au cours d'un lever de charge de type « développer-couché ».

La figure 2 illustre de manière schématique une évolution possible de la force F, de la vitesse V et de la puissance P déployée par un athlète qui soulève différentes charges de masse m variable.

La figure 3 illustre de manière schématique la plage de vitesse autour de la vitesse de consigne fixée.

La figure 4 est un diagramme de flux illustrant différentes étapes d'un exemple de procédé.

La figure 5 est une vue de dessus d'un exemple d'accéléromètre portable.

La figure 6 est un schéma-bloc d'un exemple d'accéléromètre portable.

### Exemple(s) de mode de réalisation de l'invention

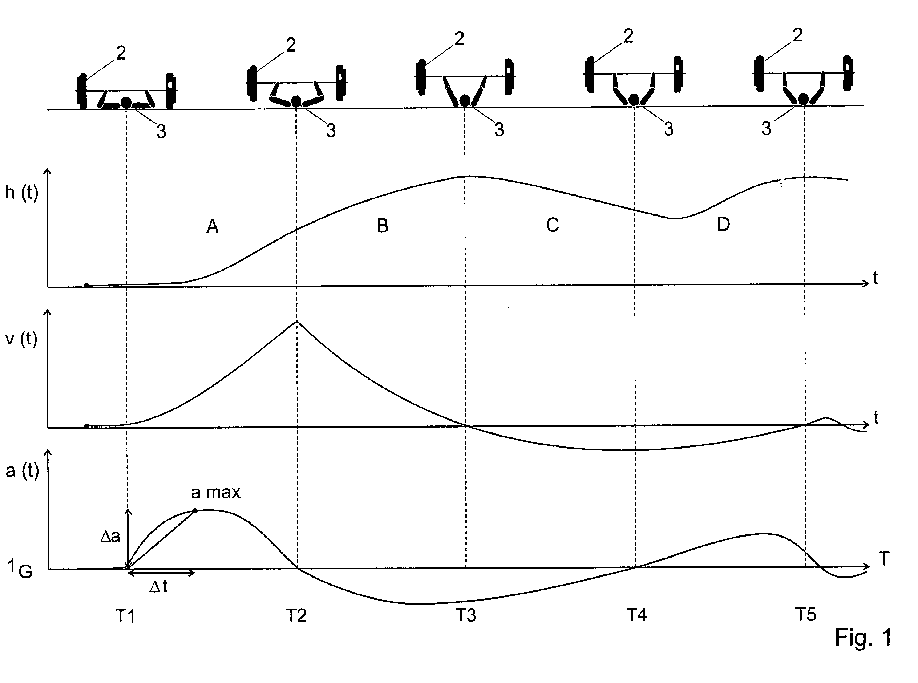

La figure 1 illustre l'évolution de différents paramètres cinématiques au cours d'un mouvement de lever de charge de type « développer-couché ». Ce mouvement, très utilisé en musculation, consiste à soulever une charge 2 avec les deux bras, à partir d'une position couchée sur le dos. La charge est soulevée le plus haut possible en combinant une adduction de l'épaule et une extension du coude. La ligne supérieure de la figure 1 illustre cinq phases du mouvement. L'exercice démarre en T1, dans la position initiale illustrée sur la première image à gauche de la figure 1. La charge est à son point (h(t) = 0) le plus bas, les coudes de l'athlète 3 sont pliés.

Au cours de la phase A, entre les instants T1 et T2, l'athlète 3 soulève la charge dont la vitesse v(t) et la hauteur h(t) augmentent constamment, comme indiqué sur les graphes correspondants. La force de poussée (proportionnelle à l'accélération a(t)) déployée au cours de cette phase est maximale et les bras s'étendent.

Au cours de la phase B, entre les instants T2 et T3, la poussée se poursuit, mais la vitesse de lever v(t) diminue ; l'accélération a(t) devient donc inférieure à 1G (gravitation terrestre), comme on le voit sur le graphe inférieur de la figure 1. La hauteur de la charge est maximale au point T3.

L'athlète 3 relâche ensuite son effort au cours de la phase C entre les instants-clés T3 et T4. La charge redescend légèrement, en sorte que sa vitesse devient négative. Cette étape est suivie par une phase D de stabilisation, entre les instants T4 et T5, au cours de laquelle l'athlète maintient ses bras en extension, mais tend à abaisser les épaules. L'accélération subie par la charge 2 au cours de cette phase tend progressivement vers 1 G.

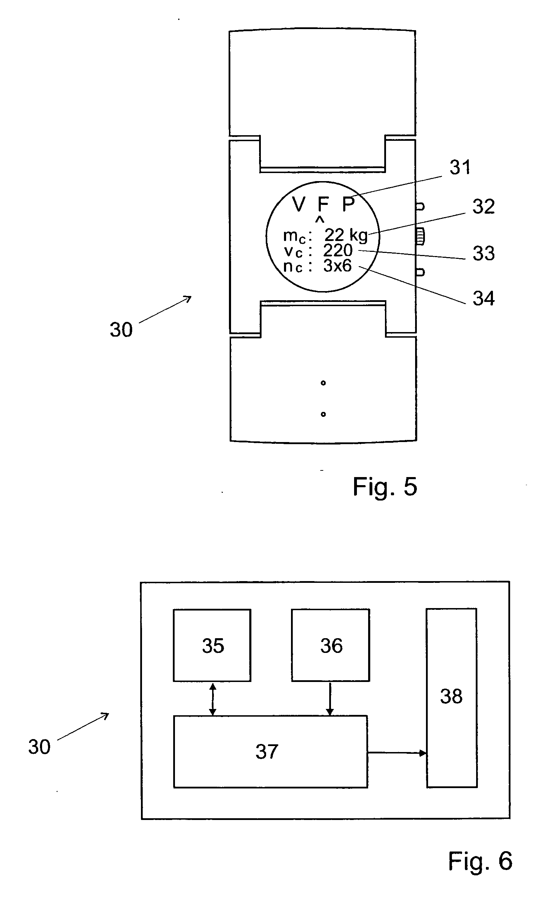

L'accélération subie par la masse soulevée peut être mesurée avec un accéléromètre lié à la masse en déplacement, par exemple un accéléromètre triaxial fixé à la masse 2 par un velcro, par exemple un accéléromètre monté dans un boîtier similaire à celui décrit dans la demande internationale WO07EP052413. Dans une variante préférentielle, l'accélération est mesurée à l'aide d'un accéléromètre 30 monté dans une montre-bracelet portée par l'utilisateur, tel qu'illustrée sur la figure 5 ; le poignet de l'athlète suit en effet au moins approximativement les déplacements de la masse soulevée par l'athlète, et est soumis sensiblement aux mêmes accélérations.

A part le développer-coucher, le dispositif et le procédé de l'invention peuvent également être utilisés avec d'autres types d'exercices pour entraîner la capacité musculaire d'autres groupes musculaires, à l'aide d'une répétition de déplacements rapides d'une masse connue. Par exemple, l'invention s'applique à d'autres exercices de levers de charge, de déplacements de charges à l'encontre d'une force, de sauts, etc. Plus généralement, l'invention s'applique à différents types d'entraînement de la capacité musculaire par une série de déplacements brefs d'une masse connue, à chaque fois que l'accélération subie par cette masse peut être mesurée avec un accéléromètre portable. Le procédé peut impliquer une sélection par l'utilisateur du type d'exercice effectué, de manière à configurer l'accéléromètre.

Un exemple d'accéléromètre 30 est illustré à l'aide du schéma-bloc de la figure 6. Cet accéléromètre peut être intégré dans la montre-bracelet de la figure 5 ou dans un autre boîtier portatif. Il comporte de préférence un capteur d'accélération 36, de préférence un capteur triaxial permettant de déterminer par projection l'accélération selon la trajectoire de déplacement, par exemple l'accélération selon la direction verticale. Un accéléromètre triaxial est particulièrement avantageux lorsque le capteur est intégré dans un bracelet, qui peut se retrouver dans différentes positions selon le mouvement. Le capteur 36 peut aussi intégrer un gyroscope, par exemple un gyroscope triaxial, bien que cela renchérisse le dispositif et ne soit pas requis pour la présente invention.

Les valeurs successives d'accélération fournies par l'accéléromètre 36 sont transmises à un microcontrôleur 37 exécutant un programme mémorisé dans une mémoire 35. Les données d'accélération, et/ou des valeurs dérivées de ces données, peuvent aussi être stockées dans cette mémoire 35. Le microcontrôleur 37 peut aussi restituer des signaux visuels et/ou acoustiques sur des moyens de restitution 38, par exemple un écran à cristaux liquides et/ou un haut-parleur. Il est aussi possible de transmettre ces données à un système de traitement à distance, par exemple via une interface filaire de type USB par exemple, ou sans fil de type Bluetooth ou Zigbee par exemple. L'accéléromètre 30 est de préférence autonome électriquement et portable, par exemple au poignet.

Le microcontrôleur 37 permet ainsi d'acquérir une série de valeurs d'accélération lors de l'entraînement, puis de calculer lors de l'entraînement, et/ou au terme de cet entraînement, d'autres valeurs dérivées de cette accélération. Les valeurs calculées par le microcontrôleur 37 à partir de la série de données d'accélération peuvent inclure par exemple la vitesse de déplacement de la masse, sa hauteur, l'énergie dépensée par l'athlète, sa puissance, etc.

En se référant à nouveau à la figure 1, la vitesse calculée et/ou stockée peut correspondre par exemple à la vitesse maximale au cours du déplacement, à la vitesse moyenne, à la vitesse instantanée lors de certains instants clés, etc. Dans un mode de réalisation, l'accéléromètre détermine uniquement le temps entre deux instants clés, par exemple le temps de vol lors d'un saut ou la durée des phases A et B de la figure 1 lors d'un développé-couché. Dans ce cas, la consigne de vitesse peut correspondre à une durée, la longueur du déplacement étant implicite ou supposée constante.

Avant de commencer une séance d'entraînement d'un groupe musculaire, un athlète doit sélectionner la masse à déplacer ainsi que la vitesse à laquelle il veut la déplacer. Il peut aussi sélectionner le nombre de déplacements ou de séries de déplacements en vue d'atteindre ses objectifs, ou une durée d'entraînement, ou encore décider d'interrompre l'entraînement manuellement au cours de l'exercice. Selon l'invention, ces consignes traditionnellement fixées empiriquement ou par un entraîneur, sont choisies par l'accéléromètre portable revendiqué, qui permet aussi d'aider l'athlète à les suivre. De préférence, ces consignes sont choisies sur la base de mesures effectuées pendant un test initial avec l'accéléromètre, et en indiquant le type d'entraînement choisi.

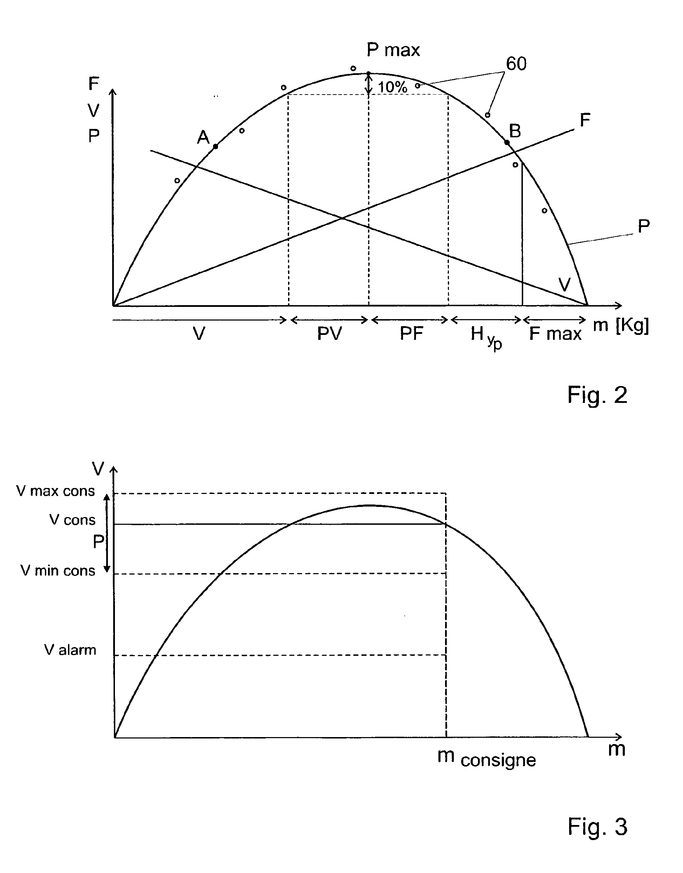

La figure 2 illustre de manière schématique une évolution possible de la force F, de la vitesse V et de la puissance P déployée par un athlète 3 qui soulève différentes charges 2 de masse m variable. La force gravitationnelle F = m X g exercée sur la charge déployée augmente linéairement avec la masse de la charge. En revanche, la vitesse de lever V diminue avec l'augmentation de la masse soulevée ; l'athlète soulève plus rapidement des masses légères.

La puissance P au cours de l'effort passe donc par un optimum pour une valeur donnée de la masse m soulevée, comme indiqué dans la demande susmentionnée WO07107491. La relation P = f(m) propre à un athlète peut être obtenue par exemple par des calculs d'interpolation et/ou d'extrapolation à partir d'une série de mesures 60 (profil) effectuées en faisant soulever à l'athlète des charges variables. En pratique, 4 à 8 mesures avec des charges différentes permettent de déterminer le profil musculaire d'un athlète avec une précision suffisante.

Cette courbe de puissance personnalisée permet de déterminer la charge avec laquelle l'athlète doit s'entraîner pour obtenir différents types de résultats. Un gain de puissance maximal peut être obtenu en s'entraînant avec la valeur de la masse correspondant à Pmax. Un athlète souhaitant améliorer sa vitesse s'entraînera de préférence dans le domaine d'entraînement V, c'est-à-dire avec une masse légère soulevée avec une puissance inférieure à la puissance maximale. Le domaine PV (Puissance et Vitesse) correspond à des masses plus légères que la masse optimale et nécessitant une puissance proche de la puissance maximale. PF (Puissance Force) nécessite aussi une puissance importante proche de la puissance maximale, mais avec des masses plus lourdes. Une hypertrophie musculaire pour des adeptes du bodybuilding sera obtenue en soulevant lentement des masses lourdes (gamme de valeurs Hyp). La gamme Fₘₐₓ au-delà de Hyp, correspond à des masses extrêmement lourdes soulevées très lentement, pour l'entraînement en force pure.

On voit ainsi que la connaissance du profil musculaire d'un athlète permet de déterminer la masse à soulever, et la vitesse de déplacement de cette masse, en vue d'atteindre les objectifs qualitatifs de l'athlète. En connaissant ces objectifs qualitatifs (c'est-à-dire le type d'entraînement qu'il souhaite effectuer, dans le but d'améliorer sa vitesse, sa puissance ou sa force, etc), ce profil permet de déterminer des consignes d'entraînement personnalisées, c'est-à-dire les valeurs de masse et de vitesse qui permettent d'effectuer un entraînement dans la zone choisie (V, PV, PF, Hyp).

Afin de déterminer son profil musculaire, et donc de choisir des consignes pour la masse et la vitesse de déplacement, un athlète peut effectuer un test, par exemple une série de levers ou de sauts, de préférence avec des charges variables, et en mesurant avec un accéléromètre la vitesse de la masse lors de cet exercice. Un tel test doit être effectué avant l'entraînement, puis répété régulièrement afin de mesurer les progrès effectués et d'adapter le plan d'entraînement en conséquence. De nombreux utilisateurs jugent cependant le temps et l'effort consacré au test (qui s'ajoutent au temps et à l'effort de l'entraînement proprement dit) contraignant. Par ailleurs, même un test de profil musculaire hebdomadaire ne tient pas compte des variations de forme physique de chaque personne au fil de la semaine.

Afin d'éviter ces inconvénients, dans un mode de réalisation avantageux de l'invention, les valeurs de consigne sont déterminées ou adaptées en fonction de mesures de valeurs d'accélération effectuées tout au long de l'entraînement, au lieu de les déterminer à l'aide d'une séance de test supplémentaire. Ces consignes peuvent avantageusement être déterminées avec un accéléromètre 30 tel que celui-illustré sur la figure 5, et intégré dans une montre-bracelet portée par l'athlète.

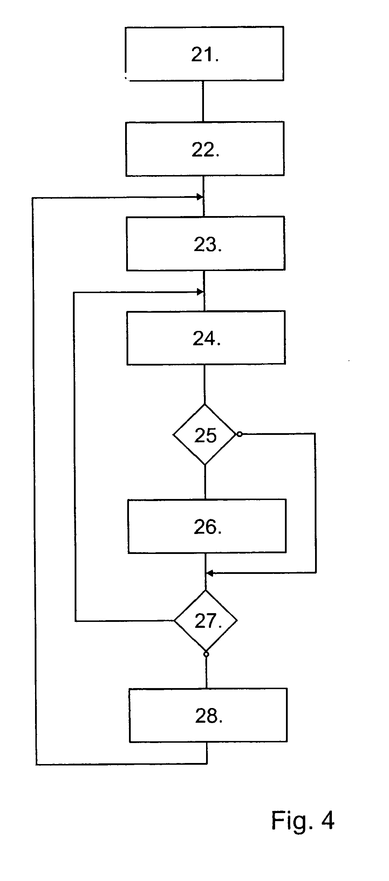

La figure 4 illustre un exemple de procédé pour déterminer et contrôler des consignes d'entraînement musculaire, c'est-à-dire des valeurs pour la masse à soulever m_{consigne}, pour la vitesse de déplacement conseillée de cette masse V_{consigne} et/ou pour le nombre n_{consigne} de déplacements ou de séries de déplacements à effectuer lors de l'entraînement. Pour certains exercices, par exemple des sauts, la masse m peut être fixe et correspondre par exemple à celle de l'athlète.

Au cours des étapes 21-23, le dispositif 30 détermine tout d'abord des valeurs de consignes initiales pour vitesse de déplacement, pour la masse qui doit être déplacée à cette vitesse, et éventuellement pour le nombre de déplacements à effectuer. Dans un mode de réalisation, ces valeurs initiales sont présélectionnées dans le dispositif, et par exemple identiques pour tous les utilisateurs, ou dépendent seulement de paramètres généraux introduits par l'utilisateur tel que son âge, son poids, son sexe, son niveau sportif (débutant, avancé, etc.). Dans une autre variante, l'utilisateur peut introduire dans l'accéléromètre des valeurs de consigne initiales qui ont par exemple été déterminées avec un autre accéléromètre, ou suggérées par un entraîneur.

Dans un mode de réalisation préférentiel, ces consignes initiales sont déterminées par l'accéléromètre 30 en faisant effectuer à l'athlète un test de profil musculaire 21 comprenant un nombre limité de déplacements de masses variables. La vitesse de déplacement de ces différentes masses est mesurée par l'accéléromètre 30 ; le microcontrôleur 37 détermine ensuite par interpolation et extrapolation un profil musculaire complet de l'athlète, et/ou détermine en fonction de ces valeurs d'accélération mesurée des valeurs de consigne initiales.

Dans une étape 22, l'athlète introduit dans l'accéléromètre 30 ses objectifs qualitatifs, par exemple s'il souhaite exercer sa vitesse, sa puissance, sa force etc. Ce choix peut par exemple être effectué en sélectionnant à l'aide des organes de commande de la montre une valeur dans une liste 31 d'objectifs prédéfinis. L'accéléromètre 30 peut aussi suggérer un choix, par exemple un choix qui dépende des résultats de mesure au cours du test 21 ou au cours d'entraînements précédents. Dans une variante, le choix est introduit en indiquant le sport auquel l'athlète souhaite se consacrer.

L'accéléromètre 30 calcule ensuite au cours de l'étape 23 une valeur de consigne V_{consigne} pour la vitesse, et éventuellement une valeur de consigne m_{consigne} pour la masse ainsi qu'une consigne n_{consigne} concernant le nombre de déplacements à effectuer afin d'atteindre ses objectifs qualitatifs, en tenant compte du profil musculaire déterminé au cours de l'étape 21 et/ou d'entraînements précédents.

Dans un mode de réalisation, ces valeurs de consigne sont affichées ou restituées par l'accéléromètre, par exemple à l'aide d'un écran à cristaux liquides (champs 32-34).

Lorsque ces valeurs de consigne initiales ont été déterminées, l'athlète s'entraîne ensuite au cours de l'étape 24 en déplaçant la masse de consigne m_{consigne} déterminée, à la vitesse de consigne V_{consigne} prescrite, pendant la durée prescrite, le nombre de fois prescrit, ou jusqu'à la fin de l'entraînement. Typiquement, le nombre de déplacements au cours d'une séance d'entraînement est beaucoup plus important que lors du test initial 21. Le test de profil initial comporte par exemple 4 à 8 mouvements, éventuellement avec des charges différentes, alors qu'une séance d'entraînement comporte par exemple 3 séries de douze déplacements avec une masse fixe, ou avec une masse qui change uniquement entre les différentes séries.

Au cours de cet entraînement 24, l'accéléromètre 30 mesure l'accélération subie par la masse 2 en déplacement, et détermine en temps réel la vitesse de déplacement ainsi qu'éventuellement d'autres valeurs dépendant de cette accélération. L'accéléromètre 30 peut aussi contrôler le nombre de déplacements et de séries de déplacement effectués, comme on le verra plus loin.

Lorsque l'athlète déplace la masse 2 trop vite ou trop lentement, c'est-à-dire en dehors d'une plage p de vitesse de consigne (Figure 3), l'accéléromètre détecte cet écart (étape 25) puis génère au cours de l'étape 26 une alarme pour demander à l'athlète de corriger cette vitesse. Dans un mode de réalisation préférentiel, différents signaux d'alarme sont générés sous forme de signaux acoustiques. Par exemple, une suite de bip-bip-bip rapides et aigus peut être générée en cas de déplacement trop rapide, et des BUUP---BUUP plus graves et plus lents en cas de déplacement en dessous de V_{min_consigne}. Cependant, l'exercice et la mesure de l'accélération ne sont pas interrompus en cas de dépassement peu important de la plage de vitesse de consigne p. L'accéléromètre 30 permet ainsi à l'athlète de s'assurer qu'il est bien en train de s'entraîner à la vitesse de consigne recommandée en fonction de ses objectifs qualitatifs.

En cas de dépassement de vitesse important, par exemple lorsque l'athlète ne parvient pas à déplacer la masse de consigne au-dessus de la vitesse Vₐₗₐᵣₘ très basse, un signal d'alarme différent, par exemple un signal d'alarme strident, peut être généré pour demander à l'athlète d'interrompre immédiatement l'entraînement. L'accéléromètre 30 sert ainsi de dispositif de sécurité pour empêcher les blessures si l'athlète ne parvient pratiquement plus à soulever les masses prescrites.

D'autres signaux peuvent être générés tout au long de l'entraînement par l'accéléromètre, y compris des signaux visuels affichés sur le dispositif, et/ou des messages vocaux générés par exemple par un synthétiseur vocal.

Au cours de l'étape 27, l'accéléromètre vérifie si l'entraînement est fini. Le procédé retourne à l'étape 24 tant que l'entraînement n'est pas fini. Si le test est fini, par exemple lorsque l'athlète a effectué le nombre de déplacements n_{consigne} prévu en fonction de ses objectifs qualitatifs, ou dans les autres cas d'interruption du test, l'accéléromètre génère de préférence un autre signal acoustique. Il sert ainsi également de compteur de déplacements et permet par exemple à l'athlète, au cours de l'entraînement, de savoir immédiatement que le nombre de déplacements n_{consigne} prescrit a été effectué. Il est également possible de contrôler le rythme de l'athlète au cours de l'entraînement, et de générer un signal au début de chaque déplacement, ou en cas de pause dépassant une durée prédéterminée.

Dans un mode de réalisation préférentiel, l'accéléromètre continue d'acquérir des mesures même si l'athlète poursuit son effort au-delà du nombre de déplacements n_{consigne} prescrits. La fin de l'acquisition intervient dans ce cas lorsque l'utilisateur sélectionne une touche à cet effet, ou si l'accéléromètre détecte une longue période d'immobilité ou un déplacement qui ne correspond pas à l'exercice attendu (par exemple si l'athlète se relève).

L'accéléromètre passe alors à l'étape 28 au cours duquel des résultats de mesures prises au cours du test, ou des valeurs calculées à partir de ces valeurs, sont de préférence affichés. L'accéléromètre peut par exemple afficher le nombre de déplacement effectués, l'énergie consommée, la puissance maximale déployée, etc. L'accéléromètre retourne ensuite à l'étape 23 au cours de laquelle de nouvelles valeurs de consigne pour la vitesse, et éventuellement pour la masse et/ou le nombre de déplacements, sont calculées en fonction de la série de valeurs d'accélération lors de l'entraînement. Ces nouvelles valeurs de consigne sont de préférence affichées ou autrement restituées à l'utilisateur, et seront utilisées pour les prochains entraînements.

L'accéléromètre permet ainsi d'effectuer un suivi constant des progrès effectués par l'athlète, et d'adapter au terme de chaque entraînement (ou même pendant cet entraînement) les valeurs de consigne en fonction des mesures d'accélération mesurées et de la vitesse calculée à partir de cette accélération. Par exemple, si le microcontrôleur 37 observe que l'utilisateur a de la difficulté à déplacer la masse de consigne à la vitesse de consigne prescrite, il peut réduire la vitesse de consigne et/ou la masse de consigne et/ou le nombre de déplacements requis. Inversement, si l'utilisateur tend à déplacer cette masse plus rapidement que la vitesse de consigne optimale, l'accéléromètre pourra préconiser un entraînement avec des masses plus lourdes, des déplacements plus rapides, et/ou des séries plus importantes.

Une adaptation des valeurs de consigne peut être effectuée même si l'utilisateur déplace constamment la masse de consigne à l'intérieur de la plage de vitesse p entre V_{max-consigne} et V_{min_consigne}. Par exemple, une vitesse de déplacement constamment en dessus de la vitesse de consigne, bien que toujours dans la plage p, ne génère aucune alarme au cours de l'entraînement, mais peut néanmoins provoquer une modification des valeurs de consigne pour les entraînements futurs.

L'adaptation des valeurs de consigne peut aussi dépendre de l'évolution du suivi des consignes au cours d'une séance d'entraînement. Par exemple, le microprocesseur 37 peut détecter des situations dans lesquelles l'athlète parvient sans difficulté à déplacer les masses requises à la vitesse de consigne au début de l'entraînement, mais se fatigue rapidement et se montre incapable d'appliquer cette vitesse lors des derniers déplacements. Il est aussi possible de réagir à la longueur des pauses prises par l'athlète entre deux déplacements successifs de la masse 2, indépendamment de la vitesse à laquelle cette masse est déplacée.

Même si l'athlète s'entraîne avec une masse de consigne m_{consigne}, les valeurs d'accélération mesurées lors de l'entraînement peuvent influencer les consignes de vitesse et/ou de nombre de déplacements appliquées à des entraînements ultérieurs avec une autre masse. Il en effet probable qu'un athlète qui a amélioré significativement ses performances dans la zone d'entraînement PV (puissance-vitesse) de la figure 2 constatera également un progrès s'il s'entraîne dans la zone PF (puissance-force) ou dans la zone F (Force). Les mesures de vitesse effectuées avec une masse donnée ont donc une influence sur tout le profil musculaire de l'athlète, même pour d'autres valeurs de masse.

Les valeurs de consigne peuvent aussi dépendre des progrès effectués par l'athlète au cours de plusieurs séances consécutives. Par exemple, le microprocesseur peut tenir compte des progrès rapides effectués par un athlète au début d'un plan d'entraînement, puis détecter un plafonnement des performances, voire une fatigue de l'athlète, lors des entraînements suivants. Dans ce cas, l'accéléromètre 30 peut par exemple proposer des entraînements différents ou des séances de récupération afin de tenir compte de cette stagnation. Dans ce but, l'accéléromètre 30 peut stocker les valeurs de consigne successivement proposées lors de différents entraînements, en liaison avec une date et une heure d'entraînement (fréquence d'entraînement), ainsi que les résultats (par exemple la vitesse moyenne, médiane, minimale et/ou maximale) obtenus lors de chaque entraînement avec ces valeurs de consigne.

L'accéléromètre 30 peut aussi proposer et contrôler le suivi de plans d'entraînements comportant plusieurs séances espacées par plusieurs heures ou plusieurs jours. Par exemple, en fonction des objectifs qualitatifs et/ou quantitatifs de l'athlète, l'accéléromètre peut proposer plusieurs entraînements par semaine, avec des masses, des vitesses et/ou des nombres de déplacement différents selon les séances. Ce plan est adapté lorsque l'athlète ne le suit pas, par exemple s'il manque une séance, s'il n'effectue pas les séances aux jours prévus, ou ne parvient pas à déplacer les masses prévues aux vitesses fixées, ou inversement si les progrès de l'athlète sont plus rapides que prévus. Dans ce cas, l'accéléromètre peut proposer de supprimer, rajouter ou déplacer des séances d'entraînement prévues dans un plan d'entraînement.

Le contrôle de la vitesse de déplacement de la masse tout au long de l'entraînement permet de prévoir de nouveaux plans d'entraînements difficiles à mettre en oeuvre sans ce contrôle. Par exemple, des plans d'entraînement comprenant des déplacements de masses variables (par exemple croissantes) à vitesse constante peuvent être proposés et contrôlés avec cet accéléromètre.

L'accéléromètre de l'invention permet ainsi de planifier des entraînements en fixant la vitesse comme consigne d'entraînement- au lieu de la puissance comme on le fait lors d'entraînements conventionnels. Les points A et B sur le graphe de la figure 2 correspondent à des déplacements à puissance égale - la vitesse de déplacement, ainsi que la masse, varient cependant de manière importante entre ces deux points, ainsi que les effets de cet entraînement sur l'athlète. Dans ce cas, le choix entre un entraînement avec les valeurs de consigne correspondant au point A ou B dépend uniquement des objectifs qualitatifs de l'athlète.

Comme indiqué plus haut, le choix des valeurs de consigne dépend en outre du type d'exercice effectué par l'athlète. L'accéléromètre permet de préférence à l'utilisateur de choisir un type d'exercice parmi une liste d'exercices disponibles, ou de définir son propre exercice. Les recommandations et le calcul de valeurs de consigne dépendent alors du type d'exercice sélectionné ou introduit.

## Revendications

1. Procédé permettant à un athlète (3) effectuant un entraînement musculaire de déterminer une consigne pour la vitesse de déplacement d'une masse (2), comprenant les étapes suivantes :
1) Détermination d'au moins une vitesse de consigne initiale à laquelle ladite masse doit être déplacée,
2) Introduction par l'athlète (3) dans un accéléromètre portable (30) d'un objectif qualitatif (V, PV, PF, Hyp, F) pour l'entraînement,
3) Lors de l'entraînement de l'athlète, mesure d'une série de valeurs d'accélération (a(n)) à l'aide du dit accéléromètre portable (30) suivant les déplacements de ladite masse ;
4) En fonction de ladite série de valeurs d'accélérations, calcul par ledit accéléromètre portable d'au moins une vitesse de consigne modifiée, au moins une valeur de consigne calculée par ledit accéléromètre dépendant dudit objectif qualitatif.

2. Le procédé de la revendication 1, dans lequel ledit accéléromètre permet de déterminer une consigne pour la masse à déplacer en fonction de mesures d'accélération effectuées lors de l'entraînement.

3. Le procédé de l'une des revendications 1 ou 2, dans lequel ledit accéléromètre permet de déterminer une consigne pour le nombre de déplacements et/ou de séries de déplacements de la masse à déplacer au cours d'un entraînement, en fonction-de mesures d'accélération effectuées lors de l'entraînement.

4. Le procédé de l'une des revendications 1 à 3, dans lequel ledit accéléromètre permet de déterminer un plan d'entraînement définissant des consignes relatives à plusieurs séances d'entraînement distinctes, en fonction de mesures d'accélération effectuées lors de l'entraînement.

5. Le procédé de l'une des revendications 1 à 4, dans lequel l'accéléromètre portable restitue à l'athlète un signal au cours de l'entraînement pour l'informer que la vitesse de déplacement de ladite masse est en dehors d'une plage de vitesse (p) autour de la vitesse de consigne.

6. Le procédé de la revendication 5, dans lequel l'accéléromètre génère un premier signal acoustique lorsque la vitesse de déplacement de ladite masse est inférieure au minimum de ladite plage de vitesse,
et un deuxième signal acoustique lorsque la vitesse de déplacement de ladite masse est supérieure au maximum de ladite plage de vitesse.

7. Le procédé de l'une des revendications 5 à 6, dans lequel la mesure de valeurs d'accélération est poursuivie même si la vitesse de déplacement de ladite masse est en dehors de ladite plage (p) de vitesse.

8. Le procédé de l'une des revendications 1 à 7, dans lequel ledit objectif qualitatif est introduit en sélectionnant un objectif parmi une liste d'objectifs (31).

9. Le procédé de l'une des revendications 1 à 8, dans lequel l'athlète introduit un dit objectif qualitatif sélectionné parmi une liste comprenant au moins un des éléments suivants :
a) vitesse
b) puissance-vitesse
c) puissance-force
d) hypertrophie
e) force.

10. Le procédé de l'une des revendications 8 à 9, dans lequel la sélection dudit objectif modifie à la fois la consigne de vitesse et une consigne pour la masse à déplacer.

11. Le procédé de l'une des revendications 1 à 10, dans lequel ladite vitesse de consigne modifiée est calculée au terme dudit entraînement, et stockée dans ledit accéléromètre portable.

12. Le procédé de l'une des revendications 1 à 11, dans lequel ledit accéléromètre portable (30) est intégré dans une montre bracelet.

13. Accéléromètre portable (30) comportant :
Une mémoire (35) pour stocker au moins une vitesse de consigne à laquelle une masse doit être déplacée lors d'un entraînement,
Un capteur d'accélération (36) pour mesurer une série de valeurs d'accélération au cours de l'entraînement,
Un microcontrôleur (37) exécutant un programme informatique afin de calculer au moins une vitesse de consigne modifiée dépendant de ladite série de valeurs d'accélérations,
Un indicateur (38) pour afficher une liste à choix (31) permettant à l'utilisateur de sélectionner un objectif qualitatif pour l'entraînement parmi plusieurs objectifs prédéfinis,
ladite vitesse de consigne calculée par ledit programme informatique dépendant de l'objectif qualitatif sélectionné.

14. L'accéléromètre de la revendication 13, agencé pour la mise en oeuvre du procédé de l'une des revendications 1 à 12.

15. L'accéléromètre de l'une des revendications 13 ou 14, intégré dans une montre-bracelet.

## Claims

1. Method enabling an athlete (3) performing a muscular training exercise to determine a setpoint for the rate of displacement of a displaced mass (2), comprising the following steps:
1) determining at least one initial setpoint rate at which said mass has to be displaced;
2) entering, by the athlete (3), into a portable accelerometer (30) of a qualitative objective for training;
3) during the athlete's training exercise, measuring a series of acceleration values (a(n)) using said portable accelerometer (30) according to the displacements of said mass; and
4) depending on said series of acceleration values, having said portable accelerometer calculate at least one modified setpoint rate, with at least one setpoint value calculated by said accelerometer being dependent on said qualitative objective.

2. The method of claim 1, wherein said accelerometer makes it possible to determine a setpoint for the mass to be displaced according to the acceleration measurements performed during training.

3. The method of one of the claims 1 to 2, wherein said accelerometer makes it possible to determine a setpoint for the number of displacements and/or series of displacements of the mass to be displaced during training, according to the acceleration measurements performed during training.

4. The method of one of the claims 1 to 3, wherein said accelerometer makes it possible to determine a training plan defining setpoints relative to several distinct training sessions, according to the acceleration measurements performed during the training.

5. The method of one of the claims 1 to 4, wherein the portable accelerometer reproduces to the athlete a signal during training to inform him/her that the displacement rate of said mass lies outside a speed range (p) around the setpoint rate.

6. The method of claim 5, wherein said accelerometer generates a first acoustic signal when the displacement rate of said mass is lower than the minimum of said speed range,
and a second acoustic signal when the displacement rate of said mass is greater than the maximum of said speed range .

7. The method of one of the claims 5 or 6, wherein the measuring of acceleration values is continued even if the displacement rate of said mass lies outside said speed range (p).

8. The method of one of the claims 1 to 7, wherein said qualitative objective is entered by selecting an objective from a list of objectives (31).

9. The method of one of the claims 1 to 8, wherein the athlete introduces one said qualitative objective selected among a list having at least one of the following elements:
a) speed
b) power-speed
c) power-force
d) hypertrophy
e) force.

10. The method of one of the claims 8 or 9, wherein selecting said objective modifies both the speed setpoint and a setpoint for the mass to be displaced.

11. The method of one of the claims 1 to 10, wherein said modified setpoint rate is computed at the end of said training session and stored in said portable accelerometer.

12. The method of one of the claims 1 to 11, wherein said portable accelerometer (30) is integrated in a wristwatch.

13. Portable accelerometer (30), including:
a memory (35) for storing at least one setpoint rate at which a mass must be displaced during a training exercise,
an acceleration sensor (35) for measuring a series of acceleration values during training,
a microcontroller (37) executing a computer program in order to calculate at least one modified setpoint rate depending on said series of acceleration values,
an indicator for displaying a menu list (31) allowing the user to select a qualitative objective for training from among several predefined objectives,
wherein said setpoint rate computed by said computer program depends on the selective qualitative objective.

14. The accelerometer of claim 13, arranged for carrying out the method of one of the claims 1 to 12

15. The accelerometer of one of the claims 13 or 14, integrated in a wristwatch.

## Patentansprüche

1. Verfahren, das einem ein Muskeltraining ausführenden Athleten (3) erlaubt, einen Sollwert für die Rate einer Verschiebung einer verschobenen Masse (2) zu bestimmen, aufweisend die folgenden Schritte:
1) Bestimmen mindestens einer Ausgangssollwertrate, bei der die Masse verschoben werden muss;
2) Eingabe, durch den Athleten (3), eines qualitativen Trainingsziels in einen tragbaren Beschleunigungsmeter (30);
3) während der Trainingsübung des Athleten, Messen einer Serie von Beschleunigungswerten (A(N)) entsprechend der Verschiebung der Masse unter Benutzung des tragbaren Beschleunigungsmeters (30); und
4) abhängig von der Serie von Beschleunigungswerten, Berechnen durch den tragbaren Beschleunigungsmeter mindestens einen geänderten Sollwert, wobei der mindestens eine durch den tragbaren Beschleunigungsmeter berechnete Sollwert von dem qualitativen Ziel abhängig ist.

2. Das Verfahren nach Anspruch 1, wobei der Beschleunigungsmeter es erlaubt, einen Sollwert für die zu verschiebende Masse entsprechend der während des Trainings gemessenen Beschleunigungsmessungen zu bestimmen.

3. Das Verfahren nach einem der Ansprüche 1 bis 2, wobei der Beschleunigungsmeter es erlaubt, einen Sollwert für die Anzahl der Verschiebungen und/oder der Serien der während des Trainings zu verschiebende Masse entsprechend der während des Trainings gemessenen Beschleunigungsmessungen zu bestimmen.

4. Das Verfahren nach einem der Ansprüche 1 bis 3, wobei der Beschleunigungsmeter es erlaubt, einen Trainingsplan, der Sollwerte für verschiedene getrennte Trainingseinheiten definiert, entsprechend der während des Trainings gemessenen Beschleunigungsmessungen zu bestimmen.

5. Das Verfahren nach einem der Ansprüche 1 bis 4, wobei der tragbare Beschleunigungsmeter während des Trainings dem Athleten ein Signal wiedergibt, um ihn/sie zu informieren, dass die Verschiebungsrate der Masse ausserhalb eines Geschwindigkeitsbereichs (p) um die Sollwertrate liegt.

6. Das Verfahren nach Anspruch 5, wobei der Beschleunigungsmeter
ein erstes akustisches Signal erzeugt, wenn die Verschiebungsrate der Masse niedriger als das Minimum des Geschwindigkeitsbereichs ist,
ein zweites akustisches Signal erzeugt, wenn die Verschiebungsrate der Masse grösser als das Maximum des Geschwindigkeitsbereichs ist.

7. Das Verfahren nach einem der Ansprüche 5 oder 6, wobei die Beschleunigungswerte weiter gemessen werden, auch wenn die Verschiebungsrade der Masse ausserhalb der Geschwindigkeitsrate (p) liegt.

8. Das Verfahren nach einem der Ansprüche 1 bis 7, wobei das qualitative Ziel durch Auswahl eines Ziels aus einer Liste von Zielen (31) eingegeben wird.

9. Das Verfahren nach einem der Ansprüche 1 bis 8, wobei der Athlet eines der aus der Liste ausgewählten qualitativen Ziele einführt, das mindestens eines der folgenden Elemente aufweist:
a) Geschwindigkeit
b) Leistung-Geschwindigkeit
c) Leistung-Kraft
d) Hypertrophie
e) Kraft.

10. Das Verfahren nach Anspruch 8 oder 9, wobei die Auswahl der Ziele den Geschwindigkeitssollwert und den Sollwert für die zu verschiebende Masse modifiziert.

11. Das Verfahren nach einem der Ansprüche 1 bis 10, wobei die modifizierte Sollwertsrate am Ende der Trainingseinheit berechnet wird und in dem tragbaren Beschleunigungsmeter gespeichert wird.

12. Das Verfahren nach einem der Ansprüche 1 bis 11, wobei der tragbare Beschleunigungsmeter in eine Armbanduhr integriert ist.

13. Tragbarer Beschleunigungsmeter (30), aufweisend:
einen Speicher (35) zum speichern mindestens eines Sollwertsrate, bei der eine Masse während des Trainings verschoben werden muss,
einen Beschleunigungssensor (35) zum Messen einer Serie von Beschleunigungswerten während des Trainings,
einen Microsteuereinheit (37), die ein Computerprogramm ausführt, um mindestens eine modifizierte Sollwertsrate in Abhängigkeit von der Serie von Beschleunigungswerten während des Trainings berechnet,
eine Anzeigeelement zum Anzeigen einer Menüliste (31), die dem Benutzer erlaubt, eine qualitatives Ziel für das Training aus verschiedenen vordefinierten Zielen zu wählen,
wobei die durch das Computerprogramm berechnete Sollwertsrate von dem ausgewählten qualitativen Ziel abhängt.

14. Der Beschleunigungsmeter nach Anspruch 13, ausgebildet zum Ausführen des Verfahrens nach einem der Ansprüche 1 bis 12.

15. Der Beschleunigungsmeter nach Anspruch 13 oder 14, integriert in eine Armbanduhr.
